(19) Europäisches Patentamt / European Patent Office / Office européen des brevets

(11) **EP 3 251 910 A1**

(12) **EUROPEAN PATENT APPLICATION**

(43) Date of publication:
06.12.2017  Bulletin 2017/49

(21) Application number: 17158182.0

(22) Date of filing: 27.02.2017

(51) Int Cl.:
**B60W 50/00** *(2006.01)*    **B60W 40/08** *(2012.01)*
**A61B 5/00** *(2006.01)*    **B60W 50/14** *(2012.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority:  **31.05.2016  JP 2016108930**

(71) Applicant: **Kabushiki Kaisha Toshiba Minato-ku Tokyo 105-8001 (JP)**

(72) Inventors:
• **Shirahama, Hirokatsu**
  **Tokyo, Tokyo 105-8001 (JP)**
• **Kasami, Hideo**
  **Tokyo, Tokyo 105-8001 (JP)**

(74) Representative: **Moreland, David Marks & Clerk LLP Aurora 120 Bothwell Street Glasgow G2 7JS (GB)**

(54)  **AUTONOMOUS CONTROL SYSTEM, SERVER DEVICE, AND AUTONOMOUS CONTROL METHOD**

(57)    An autonomous control system (100) according to an arrangement includes a first detecting unit (11), an identifying unit (12), a first determining unit (24), a learning unit (19), a second determining unit (13) and a controlling unit (14). The first detecting unit (11) detects surrounding information of an object. The identifying unit (12) identifies identification information indicating the object from the surrounding information. The first determining unit (24) determines an increase and decrease in stress information of a user. The learning unit (19) learns correction information for correcting an operation of the object, to an operation of reducing the stress of the user. The second determining unit (13) determines a type of control relative to the object, and determines control information for specifying the operation of the object by the determined type of control, from the identification information and the correction information. The controlling unit (14) controls the object by the control information.

FIG.8

## Description

### FIELD

The present disclosure relates to an autonomous control system, a server device, and an autonomous control method.

### BACKGROUND

A conventional technology for correcting an operation such as speed of a movable body, based on a pulse rate of a person traveling on the movable body, when the movable body is operated according to an operation instruction of the person traveling on the movable body has been known.

However, in the conventional technology, it has been difficult to operate an object to be controlled that is operated by a plurality of types of controls, while autonomously adapting the operation to the user's preference.

### BRIEF DESCRIPTION OF THE DRAWINGS

FIG. 1 is a diagram illustrating an example of a device configuration of an autonomous control system according to a first arrangement;

FIG. 2 is a diagram illustrating an example of a communication frame format of an inquiry request according to the first arrangement;

FIG. 3 is a diagram illustrating an example of a communication frame format of an inquiry response according to the first arrangement;

FIG. 4 is a diagram illustrating an example of an inquiry period as well as a stress information increase and decrease according to the first arrangement;

FIG. 5A is a diagram illustrating an example of a stress information increase and decrease (in the case of the number of times) according to the first arrangement;

FIG. 5B is a diagram illustrating an example of a stress information increase and decrease (in the case of an accumulated value) according to the first arrangement;

FIG. 5C is a diagram illustrating an example of a stress information increase and decrease (in the case of an integrated value) according to the first arrangement;

FIG. 6 is a diagram illustrating an example of a hardware configuration of a learning unit according to the first arrangement;

FIG. 7 is a diagram illustrating an example of a hardware configuration of a detection unit and a calculation unit according to the first arrangement;

FIG. 8 is a flowchart illustrating an example of an autonomous control method according to the first arrangement;

FIG. 9 is a diagram illustrating an example of autonomous control (in the case of parking) according to the first arrangement;

FIG. 10 is a diagram illustrating an example of a device configuration of an autonomous control system according to a second arrangement;

FIG. 11 is a diagram illustrating an example 1 of a communication frame format of reception data according to the second arrangement;

FIG. 12 is a diagram illustrating an example 2 of the communication frame format of the reception data according to the second arrangement;

FIG. 13 is a diagram illustrating an example of a hardware configuration of an autonomous control device of the first and second arrangements;

FIG. 14 is a diagram illustrating an example of a hardware configuration of a determination device of the first and second arrangements; and

FIG. 15 is a diagram illustrating an example of a hardware configuration of a server device according to the second arrangement.

### DETAILED DESCRIPTION

An autonomous control system according to an arrangement includes a first detecting unit, an identifying unit, a first determining unit, a learning unit, a second determining unit and a controlling unit. The first detecting unit detects surrounding information of an object. The identifying unit identifies identification information indicating the object from the surrounding information. The first determining unit determines an increase and decrease in stress information of a user. The learning unit learns correction information for correcting an operation of the object, to an operation of reducing the stress of the user. The second determining unit determines a type of control relative to the object, and determines control information for specifying the operation of the object by the determined type of control, from the identification information and the correction information. The controlling unit controls the object by the control information.

Hereinafter, preferred arrangements of an autonomous control system, a server device, and an autonomous control method will be described in detail with reference to the accompanying drawings.

**First Arrangement**

First, a first arrangement will be described.

Device Configuration of Autonomous Control System

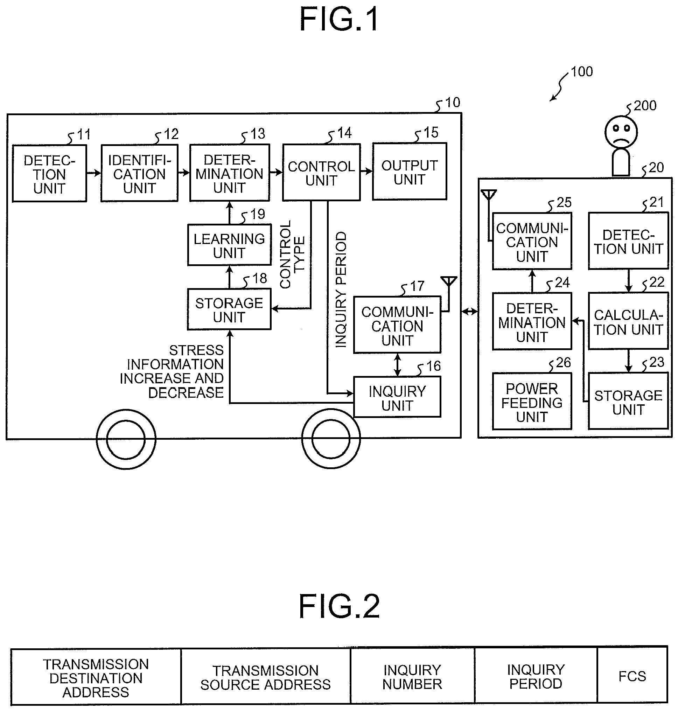

FIG. 1 is a diagram illustrating an example of a device configuration of an autonomous control system 100 of a first arrangement. The autonomous control system 100 according to the first arrangement includes an autonomous control device 10 and a determination device 20.

The autonomous control device 10 is a device that autonomously controls an operation of an object to be controlled, by a plurality of types of controls. In the first arrangement, an example in which the object to be controlled is a movable body such as an automobile will be described. In other words, an example in which the autonomous control device 10 is mounted on a movable body such as an automatic driving vehicle, and a user 200 is traveling in the movable body will be described.

The object to be controlled is not limited to the movable body in which the user 200 is traveling. The object to be controlled may also be a robot, a drone, a marine robot, a monitoring terminal, and the like. For example, the monitoring terminal is a terminal for notifying and warning the user 200, corresponding to the action of the user 200, using sound and the like.

The determination device 20 detects biological information of the user 200 of the autonomous control device 10, and determines the increase and decrease (variation amount) in stress information based on the biological information. For example, the biological information is heartbeat, an amount of perspiration, body temperature, and odor. The stress information indicates a degree of stress of the user. For example, the stress information may be indicated by a value of 256 gradations. The stress of the user 200 may be large with an increase in the value of the stress information, or the stress of the user 200 may be large with a decrease in the value of the stress information. For example, when the value of the stress information is increased, the psychological state of the user 200 is changed to an unpleasant psychological state, and when the value of the stress information is decreased, the psychological state of the user 200 is changed to a pleasant psychological state.

For example, the determination device 20 is a wearable device and an implant device. For example, the wearable device is underwear, shoes, socks, gloves, a mask, a scarf, a hat, glasses, contact lenses, a watch, and conductive clothes. The implant device is a device such as a microchip that is embedded in the user 200.

Functional Configuration of Autonomous Control Device

Next, an example of a functional configuration of the autonomous control device 10 according to the first arrangement will be described. The autonomous control device 10 according to the first arrangement includes a detection unit 11, an identification unit 12, a determination unit 13, a control unit 14, an output unit 15, an inquiry unit 16, a communication unit 17, a storage unit 18, and a learning unit 19.

The detection unit 11 detects the surrounding information of the movable body on which the autonomous control device 10 is mounted. For example, the detection unit 11 is implemented using a sensor such as a complementary metal-oxide semiconductor (CMOS) camera, a millimeter wave radar, and a laser imaging detection and ranging (LIDAR).

The identification unit 12 identifies identification information indicating an object to be identified, from the surrounding information detected by the detection unit 11. For example, the object to be identified is another vehicle in which the user 200 is not traveling, a pedestrian, an intersection, traffic lights, and a car park.

The determination unit 13 determines a control type of control relative to an object to be controlled, from identification information that is identified by the identification unit 12 and correction information that is learned by the learning unit 19. The determination unit 13 then determines control information for specifying an operation of the object to be controlled by the determined type of control.

The control type indicates the type of control of the movable body on which the autonomous control device 10 is mounted. For example, the control type includes advancing straight at a yellow traffic light, turning right at a yellow traffic light, overtaking, and parking.

For example, the control information is a control parameter including one or more parameters that are specified for each control type. For example, the control parameter includes a position parameter indicating the position of the movable body, a speed parameter indicating the speed of the movable body, and an acceleration parameter indicating the acceleration of the movable body. For example, when the control type is parking, the position parameter included in the control parameter controls the position where the movable body is to be parked.

The correction information is information for correcting the operation of the object to be controlled to an operation preferred by the user 200. For example, the correction information is a correction parameter (determination weight) including one or more parameters. In the first arrangement, the correction information is the correction parameter. For example, the correction parameter includes a parameter for correcting the position parameter described above to a position preferred by the user 200, a parameter for correcting the speed parameter described above to the speed preferred by the user 200, and a parameter for correcting the acceleration described above to the acceleration preferred by the user 200. When the determination unit 13 corrects the control parameter using the correction parameter that is learned by the learning unit 19, it is possible to autonomously adapt the control of the control unit 14 to the control preferred by the user 200.

The correction parameter is a parameter that is learned based on the changed amount of the stress information (hereinafter, referred to as a "stress information increase and decrease") of the user 200. The details of the correction parameter and a learning method of the correction parameter will be described later.

The control unit 14 controls the operation of the movable body on which the autonomous control device 10 is mounted, based on the control type and the control parameter determined by the determination unit 13. The control unit 14 also starts controlling the operation of the movable body, and enters an inquiry period during which the stress information increase and decrease of the user 200 is inquired by the control, in the inquiry unit 16. The control unit 14 also starts controlling the operation of the movable body, and enters the control type of the control, in the storage unit 18. The storage unit 18 stores the stress information increase and decrease that is determined during the inquiry period by the determination device 20, for each control type that is entered by the control unit 14, in an associated manner.

The output unit 15 is an interface for giving a warning and an instruction request, and the like, to the user 200.

Upon receiving the inquiry period from the control unit 14, the inquiry unit 16 enters an inquiry request of the stress information increase and decrease during the inquiry period, in the communication unit 17.

The communication unit 17 communicates with the other devices. A communication method performed by the communication unit 17 is optional. The communication method of the communication unit 17 in the first arrangement is a wireless communication method. For example, upon receiving an inquiry request from the inquiry unit 16, the communication unit 17 transmits the inquiry request to the determination device 20. The communication unit 17 then receives an inquiry response from the determination device 20.

FIG. 2 is a diagram illustrating an example of a communication frame format of an inquiry request according to the first arrangement. The inquiry request according to the first arrangement includes a transmission destination address, a transmission source address, an inquiry number, an inquiry period, and a frame check sequence (FCS). The transmission destination address is the address of the determination device 20. The transmission source address is the address of the autonomous control device 10. The inquiry number is a number for identifying each control performed by the control unit 14. The inquiry period is a period during which the increase and decrease in the stress information of the user 200 is inquired. The FCS is data required for detecting and correcting an error in the data included in the communication frame.

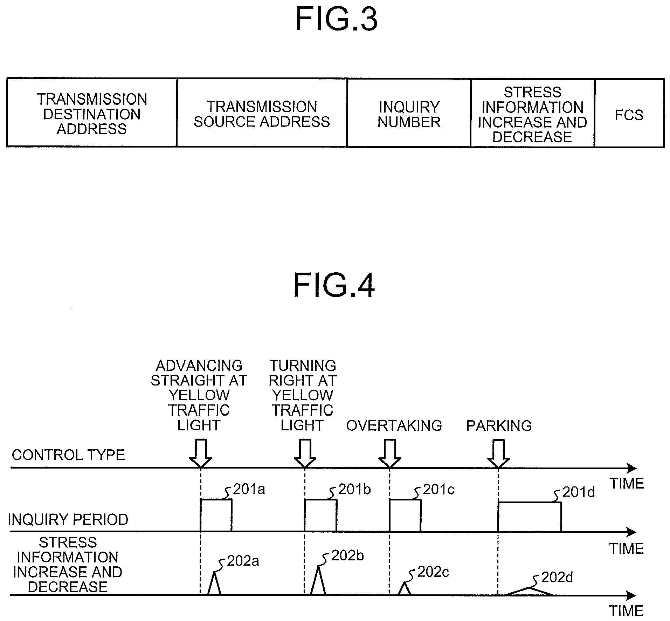

FIG. 3 is a diagram illustrating an example of a communication frame format of an inquiry response according to the first arrangement. The inquiry response according to the first arrangement includes a transmission destination address, a transmission source address, an inquiry number, a stress information increase and decrease, and an FCS. The transmission destination address is the address of the autonomous control device 10. The transmission source address is the address of the determination device 20. The inquiry number is the number for identifying each control performed by the control unit 14. The stress information increase and decrease is data indicating the increase and decrease in the stress information of the user 200. The FCS is data required for detecting and correcting an error in the data included in the communication frame.

FIG. 4 is a diagram illustrating an example of the inquiry period as well as the stress information increase and decrease according to the first arrangement.

An inquiry period 201a is an inquiry period during which the advancing straight at a yellow traffic light is controlled by the control unit 14. A stress information increase and decrease 202a indicates the increase and decrease in the stress information that is determined by the determination device 20 during the inquiry period 201a.

An inquiry period 201b is an inquiry period during which the turning right at a yellow traffic light is controlled by the control unit 14. A stress information increase and decrease 202b indicates the increase and decrease in the stress information that is determined by the determination device 20 during the inquiry period 201b.

An inquiry period 201c is an inquiry period during which the overtaking is controlled by the control unit 14. A stress information increase and decrease 202c indicates the increase and decrease in the stress information determined by the determination device 20 during the inquiry period 201c.

An inquiry period 201d is an inquiry period during which the parking is controlled by the control unit 14. A stress information increase and decrease 202d indicates the increase and decrease in the stress information that is determined by the determination device 20 during the inquiry period 201d.

The length of the inquiry periods 201a to 201d is specified for each control type. For example, the length of the inquiry period 201d is longer than the lengths of the inquiry periods 201a to 201c. This is because, the time required for controlling the parking takes longer than the time required for controlling the advancing straight at a yellow traffic light, the turning right at a yellow traffic light, and the overtaking. In other words, in the example illustrated in FIG. 4, the inquiry period 201d is specified longer than the inquiry periods 201a to 201c, so as to secure a sufficient period for determining the increase and decrease in the stress information of the user 200, from when the control of parking has started until the control of parking is finished.

Returning to FIG. 1, the inquiry unit 16 stores the stress information increase and decrease that is received from the determination device 20 by the communication unit 17, in the storage unit 18.

The storage unit 18 stores therein the control type, the controlled number of times, and the stress information increase and decrease, in an associated manner. In other words, the storage unit 18 stores therein the history of the stress information increase and decrease of the user 200, for each control type. The data format of the stress information increase and decrease is optional.

Example of Data Format of Stress Information Increase and Decrease

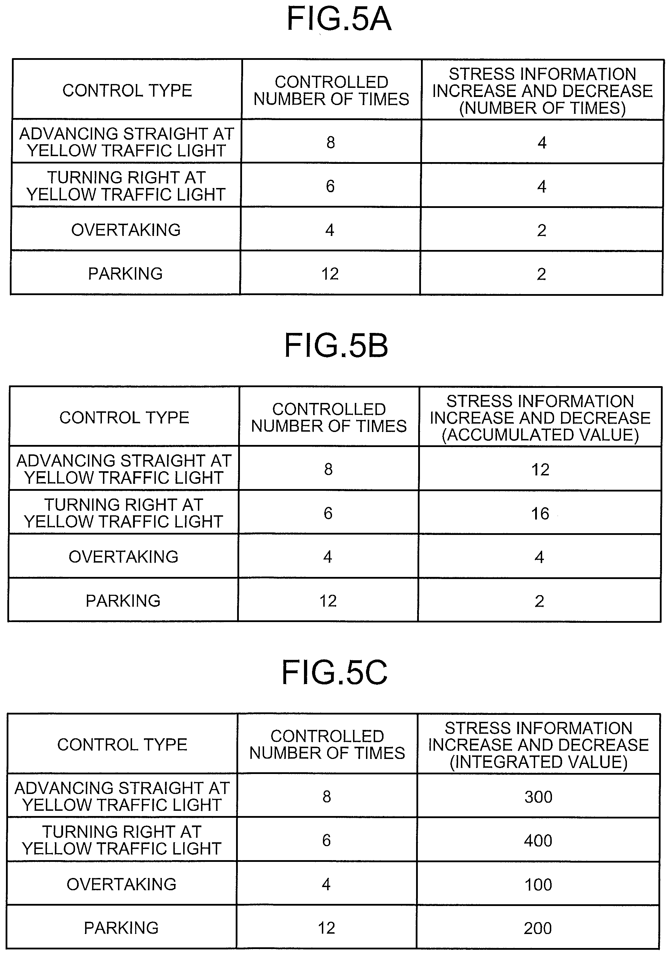

FIG. 5A is a diagram illustrating an example of a stress information increase and decrease (in the case of the number of times) according to the first arrangement. In the example in FIG. 5A, the data format of the stress information increase and decrease is the number of times when the stress information of the user 200 has exceeded a threshold during the inquiry period. For example, in the example of advancing straight at a yellow traffic light in FIG. 5A, the controlled number of times is eight times, and the stress information increase and decrease of the user 200 is four times. This indicates that among the eight times when the advancing straight at a yellow traffic light is controlled, the stress information of the user 200 has exceeded the threshold four times, while the advancing straight at a yellow traffic light is controlled during the inquiry period.

FIG. 5B is a diagram illustrating an example of a stress information increase and decrease (in the case of an accumulated value) according to the first arrangement. In the example in FIG. 5B, the data format of the stress information increase and decrease is an accumulated value of a difference in the stress information of the user 200 during the inquiry period. For example, in the example of advancing straight at a yellow traffic light in FIG. 5B, the controlled number of times is eight times, and the stress information increase and decrease of the user 200 is 12. This indicates that the value obtained by accumulating the difference between the maximum value and the minimum value of the stress information of the user 200, when the advancing straight at a yellow traffic light is controlled during the inquiry period, that is calculated for each of the eight times when the advancing straight at a yellow traffic light is controlled, is 12.

FIG. 5C is a diagram illustrating an example of a stress information increase and decrease (in the case of an integrated value) according to the first arrangement. In the example in FIG. 5C, the data format of the stress information increase and decrease is an integrated value that is obtained by integrating the function indicating the stress information of the user 200 during the inquiry period. For example, in the example of advancing straight at a yellow traffic light illustrated in FIG. 5C, the controlled number of times is eight times, and the stress information increase and decrease of the user 200 is 300. This example indicates that the sum of the integrated value of the function indicating the stress information of the user 200, in a section from the beginning to the end of the inquiry period during which the advancing straight at a yellow traffic light is controlled, that is calculated for each control of the eight times of advancing straight at a yellow right, is 300.

FIG. 5A to FIG. 5C are examples of the data formats of the stress information increase and decrease according to the first arrangement, and the stress information increase and decrease may be indicated by another data format. The storage unit 18 may also store therein a combination of a plurality of data formats of the stress information increase and decrease.

**Learning Correction Parameter**

Returning to FIG. 1, the learning unit 19 learns a correction parameter $w = \{w_0, w_1, w_2, ..., w_N\}$ for each control type, from the stress information increase and decrease of the user 200 that is stored in the storage unit 18. The learning unit 19 learns the correction parameter $w = \{w_0, w_1, w_2, ..., w_N\}$ such that the degree of unpleasantness indicated by the stress information that is determined after a part or all of the correction parameter $w = \{w_0, w_1, w_2, ..., w_N\}$ is changed is decreased (so that the degree of pleasantness is increased). The learning unit 19 then enters the correction parameter $w = \{w_0, w_1, w_2, ..., w_N\}$ in the determination unit 13, when the determination unit 13 is operated.

More specifically, first, the learning unit 19 sets a group of parameters $w_n$ ($n = 0, 1, ..., N$) capable of generating a control parameter $c = \{c_0, c_1, c_2, ..., c_N\}$ indicating the control preferred by an average user 200, as an initial value of the correction parameter.

Next, when the determination unit 13 is operated, the learning unit 19 enters $w^{ref} = \{w_0, w_1, w_2, ..., w_n^{old} + \delta,$

..., $w_N$} that has varied by a sufficiently small positive integer of $\delta$, from the current correction parameter $w^{old}$ = {$w_0$, $w_1$, $w_2$, ..., $w_n^{old}$, ..., $w_N$}, in the determination unit 13.

Next, each time a sufficient number of pieces of stress information increase and decrease $E_1$ that can be evaluated as a certain statistical amount relative to $w^{ref}$, is accumulated in the storage unit 18, the learning unit 19 updates the correction parameter $w^{old}$ = {$w_0$, $w_1$, $w_2$, ..., $w_n^{old}$, ..., $w_N$} to a new correction parameter $w^{new}$ = {$w_0$, $w_1$, $w_2$, ..., $w_n^{new}$, ..., $w_N$}, using the following formula (1).

$$W_n^{new} = W_n^{old} - \varepsilon\left(\overline{E_1} - E_0\right) \tag{1}$$

In this example, $E_0$ is a stress information increase and decrease that is determined when the control is performed using a control parameter c being generated based on the current correction parameter $w^{old}$ = {$w_0$, $w_1$, $w_2$, ..., $w_n^{old}$, ..., $w_N$}.

In addition, an $E_1$ bar is an average value of the stress information increase and decrease that is determined when the control is performed using the control parameter c being generated based on the correction parameter $w^{ref}$ = {$w_0$, $w_1$, $w_2$, ..., $w_n^{old}$ + $\delta$, ..., $w_N$}.

$\varepsilon$ is a sufficiently small positive real number.

By repeating the update using the above-described formula (1), the learning unit 19 can minimize the stress information increase and decrease of the user 200. Consequently, it is possible to adapt the control by the control unit 14, to the control that is preferred by the specific user 200 whose biological information is acquired by the determination device 20.

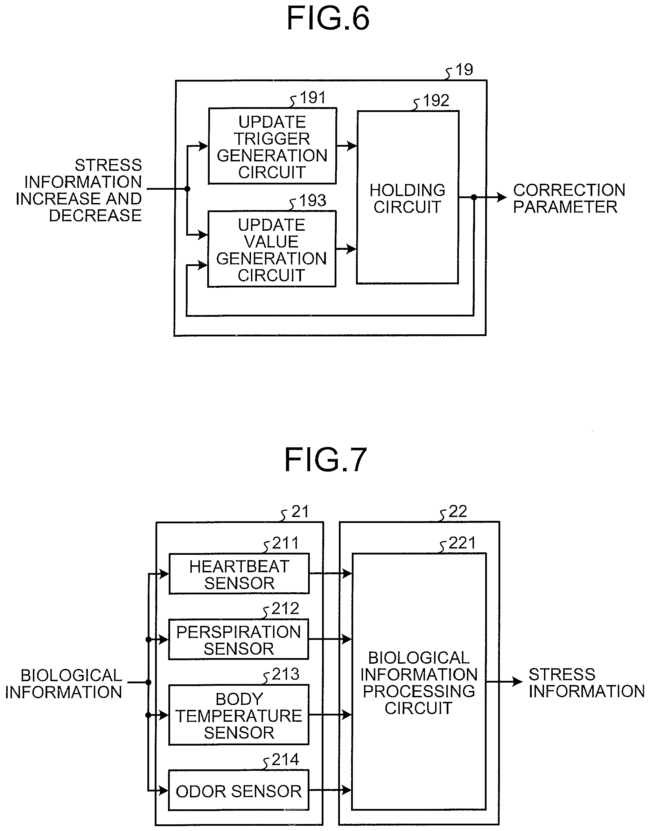

FIG. 6 is a diagram illustrating an example of a hardware configuration of the learning unit 19 according to the first arrangement. For example, the learning unit 19 according to the first arrangement is implemented using an update trigger generation circuit 191, a holding circuit 192, and an update value generation circuit 193.

The update trigger generation circuit 191 determines whether a sufficient number of pieces of stress information increase and decrease $E_1$ that can be evaluated as a certain statistical amount relative to $w^{ref}$ is accumulated in the storage unit 18. When a sufficient number of pieces of stress information increase and decrease $E_1$ that can be evaluated as a certain statistical amount is accumulated in the storage unit 18, the update trigger generation circuit 191 enters an update notification of the correction parameter w = {$w_0$, $w_1$, $w_2$, ..., $w_N$} to the holding circuit 192.

The holding circuit 192 enters the correction parameter w = {$w_0$, $w_1$, $w_2$, ..., $w_N$} that is currently held in the holding circuit 192, to the determination unit 13.

Upon receiving the update notification from the update trigger generation circuit 191, the holding circuit 192 enters the correction parameter w = {$w_0$, $w_1$, $w_2$, ..., $w_N$} that is held in the holding circuit 192, to the update value generation circuit 193. Upon receiving the updated correction parameter w = {$w_0$, $w_1$, $w_2$, ..., $w_N$} from the update value generation circuit 193, the holding circuit 192 holds the correction parameter w = {$w_0$, $w_1$, $w_2$, ..., $w_N$} in the holding circuit 192.

Upon receiving the correction parameter w = {$w_0$, $w_1$, $w_2$, ..., $w_N$} from the holding circuit 192, the update value generation circuit 193 updates the correction parameter w = {$w_0$, $w_1$, $w_2$, ..., $w_N$} using the above-described formula (1). The update value generation circuit 193 then enters the updated correction parameter w = {$w_0$, $w_1$, $w_2$, ..., $w_N$} in the holding circuit 192.

Functional Configuration of Determination Device

Next, an example of a functional configuration of the determination device 20 according to the first arrangement will be described. The determination device 20 according to the first arrangement includes a detection unit 21, a calculation unit 22, a storage unit 23, a determination unit 24, a communication unit 25, and a power feeding unit 26.

The detection unit 21 detects biological information of the user 200, and enters the biological information in the calculation unit 22. Upon receiving the biological information from the detection unit 21, the calculation unit 22 calculates stress information based on the biological information.

FIG. 7 is a diagram illustrating an example of a hardware configuration of the detection unit 21 and the calculation unit 22 according to the first arrangement.

For example, the detection unit 21 according to the first arrangement is implemented using a heartbeat sensor 211, a perspiration sensor 212, a body temperature sensor 213, and an odor sensor 214. When psychological stress including a sense of unpleasantness occurs to the user 200, it is known that increase in heartbeat, increase in perspiration, increase in body temperature, change in odor, and the like occur to the body of the user 200. The heartbeat sensor 211 detects the heartbeat rate of the user 200. The perspiration sensor 212 detects the amount of perspiration of the user 200. The body temperature sensor 213 detects the body temperature of the user 200. The odor sensor 214 detects the odor of the user 200.

For example, the calculation unit 22 according to the first arrangement is implemented by a biological information processing circuit 221. The biological information processing circuit 221 calculates a signal indicating the stress information, by combining detection values that are detected by the various sensors 211 to 214. A method for calculating stress information by the biological information processing circuit 221 is optional. For example, the biological information processing circuit 221 calculates the stress information by a process using a function for generating the stress information, a process using a table for determining the stress information, and the like. For example, when the function for generating the stress information is used, a function that is obtained by performing statistical processing on a sufficient number of pieces of experimental data (samples of a combination of the detection values detected by the various sensors 211 to 214) is used. It is also possible to use a function that is obtained by machine learning in which the function obtained by the statistical processing is used as teacher data.

Returning to FIG. 1, the calculation unit 22 stores the stress information in the storage unit 23. The determination unit 24 determines the increase and decrease in the stress information being stored in the storage unit 23.

More specifically, when the increase and decrease in the stress information is determined by the number of times (see FIG. 5A), the determination unit 24 determines whether the stress information of the user 200 has exceeded the threshold during the inquiry period. When the increase and decrease in the stress information is determined by the accumulated value (see FIG. 5B), the determination unit 24 determines (calculates) the difference between the pieces of stress information of the user 200 during the inquiry period. When the increase and decrease in the stress information is determined by the integrated value (see FIG. 5C), the determination unit 24 determines (calculates) the integrated value that is obtained by integrating the function indicating the stress information of the user 200 during the inquiry period.

The communication unit 25 communicates with the other devices. A communication method performed by the communication unit 25 is optional. The communication method of the communication unit 25 according to the first arrangement is a wireless communication method. Upon receiving an inquiry request (see FIG. 2) from the autonomous control device 10, the communication unit 25 requests the determination unit 24 to execute a determination process on the increase and decrease in the stress information during the inquiry period. The communication unit 25 then transmits an inquiry response (see FIG. 3) including the stress information increase and decrease that is determined by the determination unit 24, to the autonomous control device 10.

The power feeding unit 26 feeds power to the determination device 20 using energy harvesting. For example, the power feeding unit 26 feeds power that is generated by thermoelectric generation, piezoelectric generation, and the like, or power generated by radio frequency (RF) generator, and the like, to the determination device 20. For example, when power is fed from the RF generator, not only the inquiry request is received from the autonomous control device 10, but also power may be fed from the autonomous control device 10.

## Autonomous Control Method

Next, an example of an autonomous control method according to the first arrangement will be described.

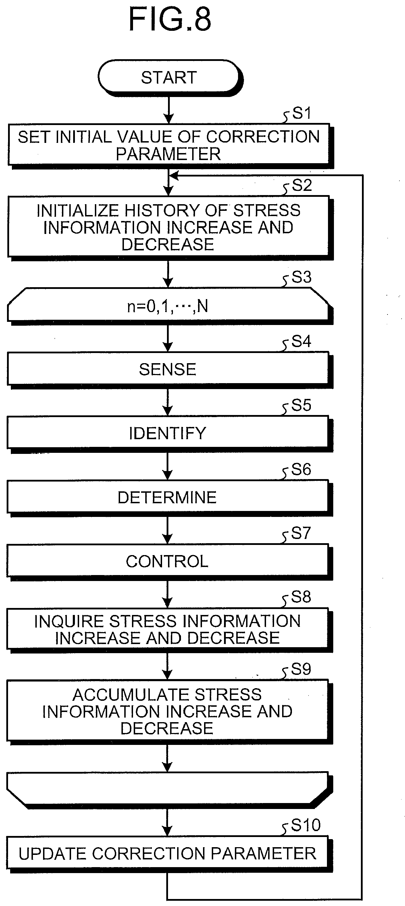

FIG. 8 is a flowchart illustrating an example of an autonomous control method according to the first arrangement. The example in FIG. 8 illustrates a method for autonomously performing a certain type of control (such as parking). The autonomous control device 10 performs a process indicated by the flow illustrated in FIG. 8, for each of the types of controls.

## Initialization Process

First, the learning unit 19 sets a group of parameters $w_n$ (n = 0, 1, ..., N) that can generate the correction parameter c = $\{c_0, c_1, c_2, ..., c_N\}$ indicating the control preferred by the average user 200, as an initial value of the correction parameter (step S1).

Next, the learning unit 19 initializes the storage unit 18 that stores therein the history of the stress information increase and decrease of the user 200 (step S2).

Next, the autonomous control device 10 repeats the processes from step S4 to step S9, on the parameter $w_n$ (n = 0, 1, ..., N) that is included in the correction parameter w = $\{w_0, w_1, w_2, ..., w_N\}$ (step S3).

## Repeating Process

The detection unit 11 detects the surrounding information of the movable body on which the autonomous control device 10 is mounted, using a sensor such as the CMOS camera, the millimeter wave radar, and the LIDAR (step S4).

Next, the identification unit 12 identifies identification information indicating an object to be identified, from the surrounding information that is detected by the detection unit 11 (step S5).

Next, the determination unit 13 determines the control type of the control relative to the object to be controlled, using the identification information that is identified by the identification unit 12 and the correction parameter that is learned by the learning unit 19, and determines a control parameter for specifying an operation of the object to be controlled, by the determined type of control (step S6). When the process at step S6 is performed for the first time, the determination unit 13 refers to the initial value of the correction parameter that is set by the process at step S1.

Next, the control unit 14 controls the operation of the movable body on which the autonomous control device 10 is mounted, based on the control type and the control parameter that are determined by the process at step S6 (step S7).

Next, the inquiry unit 16 receives the inquiry period from the control unit 14, and transmits an inquiry request of a stress information increase and decrease during the inquiry period, to the determination device 20 via the communication unit 17 (step S8).

Next, the storage unit 18 accumulates the stress information increase and decrease, by storing the stress information increase and decrease as well as the control type and the controlled number of times that are determined by the determination device 20, in an associated manner (step S9).

**Update Process**

Next, when a sufficient number of pieces of stress information increase and decrease that can be evaluated as a certain statistical amount are accumulated in the storage unit 18, for each of $w_n$ (n = 0, 1, ..., N) by repeating the processes from step S4 to step S9, the learning unit 19 updates the correction parameter $w = \{w_0, w_1, w_2, ..., w_N\}$ using the above-described formula (1) (step S10). The process then returns to step S2.

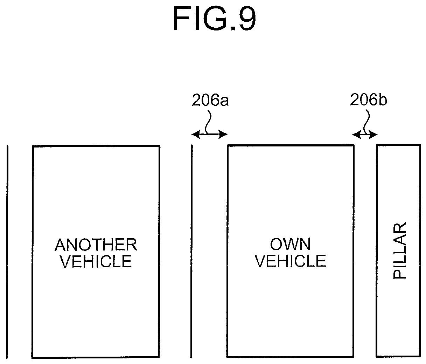

FIG. 9 is a diagram illustrating an example of an autonomous control (in the case of parking) according to the first arrangement. The example in FIG. 9 illustrates that another vehicle is parked at the left side of the own vehicle, and a pillar is located at the right side thereof. The user 200 who is seated on the driver's seat in the own right hand drive vehicle, prefers to park the vehicle by giving priority to securing boarding and alighting space 206a at the passenger's seat side, compared with boarding and alighting space 206b of the user 200. For example, by controlling the parking as illustrated in FIG. 9, the passenger in the passenger's seat can comfortably board and alight the vehicle. In addition, it is possible to reduce risk of coming into contact with a door of the other vehicle that is parked at the left side of the own vehicle.

For example, when parking is autonomously controlled as illustrated in FIG. 9, it is suitable for the user 200 and the like who often have a physically handicapped person, a child, or the like seated on the passenger's seat. When the user 200 instructs the autonomous control device 10 to autonomously control the parking, and if the stress information is increased when the boarding and alighting space 206a is narrower than the boarding and alighting space 206b, the parking can be autonomously controlled as illustrated in FIG. 9, by causing the autonomous control device 10 to learn the correction parameter so as to reduce the increase in the stress information.

In this manner, the autonomous control system 100 according to the first arrangement can determine the increase and decrease in the stress information after the control is performed, for each of the types of controls, by implementing human and machine sensing (HMS). Consequently, it is possible to operate the object to be controlled, by the types of autonomous control adapted to the preference of the user 200 who is a partner of the object to be controlled.

In the first arrangement, the determination device 20 is assumed to be a wearable device and an implant device. However, the determination device 20 may also be equipment installed on the object to be controlled. For example, when the object to be controlled is a movable body, the installed equipment may be a seat, a steering wheel, and the like.

In the first arrangement, the power feeding unit 26 feeds power to the determination device 20 using the energy harvesting. However, the power feeding unit 26 may also be a battery or the like.

As described above, in the autonomous control system 100 according to the first arrangement, the determination unit 24 determines the increase and decrease in the stress information indicating the degree of stress of the user 200, from the biological information of the user 200. The learning unit 19 learns the correction information for correcting the operation of the object to be controlled, to the operation of reducing the stress of the user 200 that is indicated by the stress information (in the explanation in the first arrangement, the correction parameter), from the increase and decrease in the stress information. The determination unit 13 determines the type of control (in the explanation in the first arrangement, the control type) relative to the object to be controlled, from the identification information and the correction information, and determines the control information (in the explanation in the first arrangement, the control parameter) for specifying the operation of the object to be controlled, by the determined type of control. The control unit 14 then controls the object to be controlled by the control information.

The autonomous control system 100 according to the first arrangement can operate the object to be controlled that is operated by the types of controls, while autonomously adapting the operation to the preference of the user 200. For example, the autonomous control system 100 according to the first arrangement can be suitably applied when the difference between the user preferences on the autonomous control is notable with the sophistication of the autonomous control, and when the user 200, who is a partner of the object to be controlled, has a possibility to feel unpleasant due to the operation that is performed by the initial setting of the object to be controlled at the time of shipping.

**Second Arrangement**

A second arrangement will now be described. In the second arrangement, the same descriptions as those according to the first arrangement are omitted, and portions different from the first arrangement will be described.

Device Configuration of Autonomous Control System

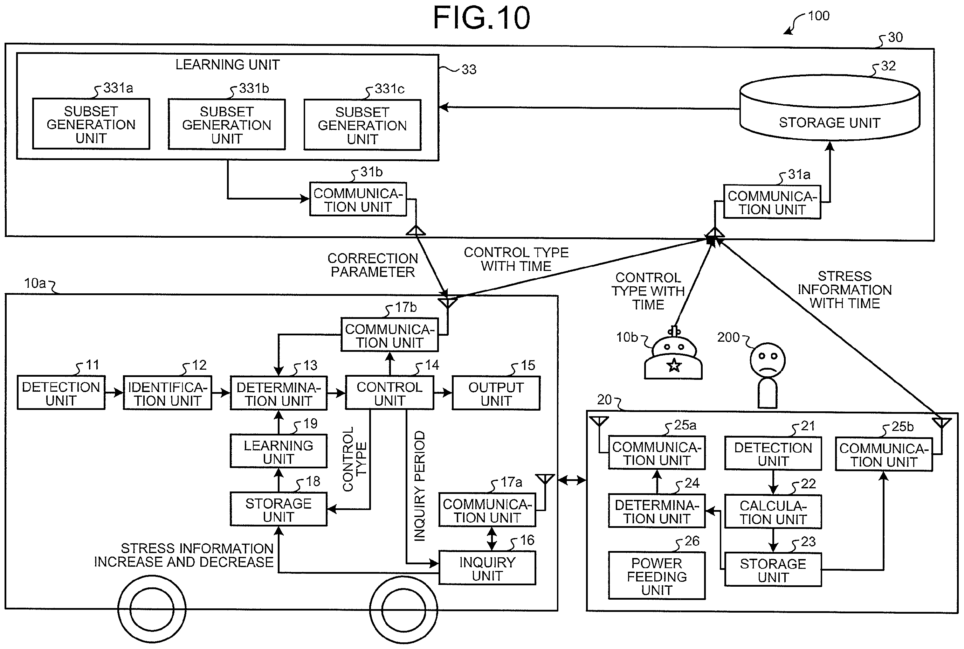

FIG. 10 is a diagram illustrating an example of a device configuration of the autonomous control system 100 of a second arrangement. The autonomous control system 100 according to the second arrangement includes an autonomous control device 10a, an autonomous control device 10b, the determination device 20, and a server device 30. In the autonomous control system 100 according to the second arrangement, the autonomous control device 10b and the server device 30 are added to the device configuration of the autonomous control system 100 according to the first arrangement.

The autonomous control device 10b is mounted on a life-supporting robot that autonomously performs the types of controls. For example, the life-supporting robot performs a service of estimating danger when an elderly person goes out, and navigating the elderly person. Hereinafter, if there is no need to distinguish between the autonomous control devices 10a and 10b, they are simply referred to as the autonomous control device 10. To simplify the explanation, there are two autonomous control devices 10 in the second arrangement. However, the number of the autonomous control device 10 to be included in the autonomous control system 100 is optional.

The server device 30 generates correction information for each type of the autonomous control devices 10, using the reception data that is received from one or more of the autonomous control devices 10 as well as one or more of the determination devices 20. For example, the correction information in the second arrangement is a correction parameter (determination weight) including one or more parameters.

The correction parameter that is generated for each type of the autonomous control devices 10 by the server device 30 can be used as an initial value of the correction parameter of the autonomous control device 10 of the same type. In addition, for example, the correction parameter that is generated for each type of the autonomous control devices 10 by the server device 30 can be used for the autonomous control device 10 that does not include the learning unit 19 in the own device.

Functional Configuration of Autonomous Control Device

Next, an example of a functional configuration of the autonomous control device 10 according to the second arrangement will be described. The autonomous control device 10a according to the second arrangement includes the detection unit 11, the identification unit 12, the determination unit 13, the control unit 14, the output unit 15, the inquiry unit 16, a communication unit 17a, a communication unit 17b, the storage unit 18, and the learning unit 19. In the autonomous control device 10 according to the second arrangement, the communication unit 17b is added to the configuration of the autonomous control device 10 in the first arrangement.

The communication unit 17b communicates with the other devices. A communication method performed by the communication unit 17b is optional. The communication method of the communication unit 17b according to the second arrangement is a wireless communication method.

For example, upon receiving a correction parameter from the server device 30, the communication unit 17b enters the correction parameter to the determination unit 13. For example, the communication unit 17b receives a correction parameter from the server device 30, by requesting an initial value of the correction parameter to the server device 30, to operate the determination unit 13 using the initial value of the correction parameter.

For example, when the control unit 14 controls the operation of the object to be controlled, the communication unit 17b transmits a control time indicating the time when the control is performed, and the control type described above, to the server device 30.

**Functional Configuration of Determination Device**

Next, an example of a functional configuration of the determination device 20 according to the second arrangement will be described. The determination device 20 according to the second arrangement includes the detection unit 21, the calculation unit 22, the storage unit 23, the determination unit 24, a communication unit 25a, a communication unit 25b, and the power feeding unit 26. In the determination device 20 according to the second arrangement, the communication unit 25b is added to the configuration of the determination device 20 in the first arrangement.

The communication unit 25b communicates with the other devices. A communication method performed by the communication unit 25b is optional. The communication method of the communication unit 25b according to the second arrangement is a wireless communication method. For example, the communication unit 25b transmits the determination time indicating the time when determination is made on the stress information, and the stress information described above, to the server device 30. For example, the communication unit 25b regularly transmits the stress information that is associated with the determination time to the server device 30, at a transmission interval such as at every minute.

**Functional Configuration of Server Device**

Next, an example of a functional configuration of the server device 30 according to the second arrangement will be described. The server device 30 according to the second arrangement includes a communication unit 31a, a communication unit 31b, a storage unit 32, and a learning unit 33.

The communication unit 31a receives the control type that is associated with the control time from one or more of the autonomous control devices 10, and receives the stress information that is associated with the determination time from one or more of the determination devices 20.

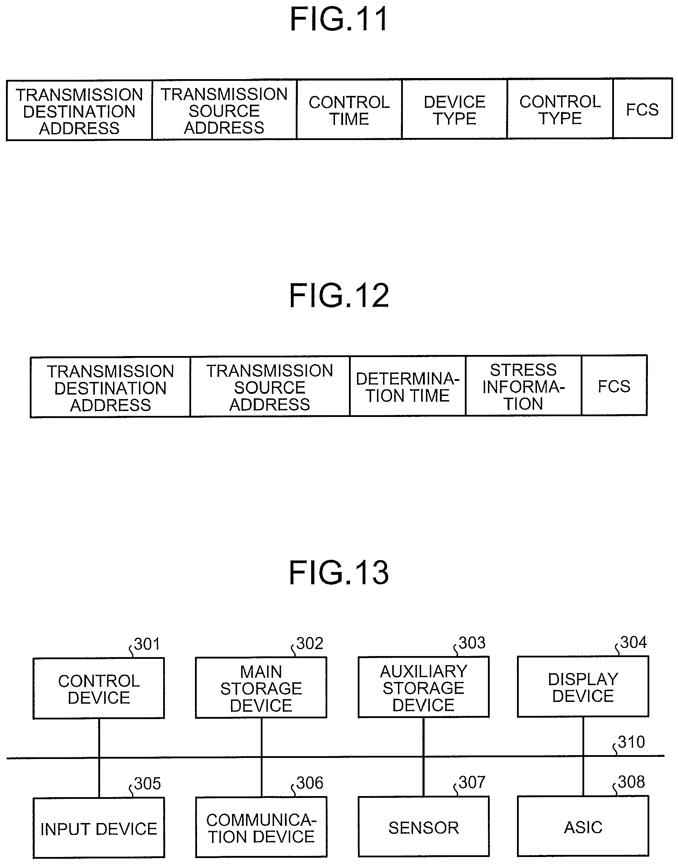

FIG. 11 is a diagram illustrating an example 1 of a communication frame format of reception data according to the second arrangement. FIG. 11 illustrates the case in which the server device 30 has received reception data from the autonomous control device 10. The transmission destination address is the address of the server device 30. The transmission source address is the address of the autonomous control device 10. The control time is the time when the autonomous control device 10 has controlled the object to be controlled. A device type is the type of the autonomous control device 10. The control type is the type of control performed by the autonomous control device 10. The FCS is data required for detecting and correcting an error in data that is included in the communication frame.

FIG. 12 is a diagram illustrating an example 2 of the communication frame format of the reception data according to the second arrangement. FIG. 12 illustrates the case in which the server device 30 has received reception data from the determination device 20. The transmission destination address is the address of the server device 30. The transmission source address is the address of the determination device 20. The determination time is the time when the stress information is determined (calculated) by the determination device 20. The stress information is a value of 256 gradations indicating the degree of stress. The FCS is data required for detecting and correcting an error in data included in the communication frame.

Returning to FIG. 10, the communication unit 31a stores the device type, the control type, and the stress information that are included in the reception data in which the difference between the control time and the determination time is equal to or less than the threshold, in the storage unit 32 in an associated manner.

The communication unit 31b transmits the correction parameter that is requested by the autonomous control device 10, among the correction parameters generated by the learning unit 33 for each combination of the device type and the control type, to the autonomous control device 10.

The storage unit 32 stores therein the control time (determination time), the device type, the control type, and the stress information, in an associated manner. For example, the storage unit 32 may also separately store therein the device type, the control type, and the stress information, using a table in which the control time, the device type, and the control type are associated with one another, and a table in which the determination time and the stress information are associated with each other.

The learning unit 33 includes a subset generation unit 331a, a subset generation unit 331b, and a subset generation unit 331c. The learning unit 33 generates a correction parameter different for each type of the autonomous control devices 10, by learning the control preferred by the user 200 for each type of the autonomous control device 10.

For example, the subset generation unit 331a learns a correction parameter when the type of the autonomous control device 10 is a movable body, for each control type. More specifically, the subset generation unit 331a reads out the history of the stress information of the user 200 that is associated for each device type indicating the movable body, from the storage unit 32 for each control type. The subset generation unit 331a then calculates the increase and decrease in the stress information for each control type. The subset generation unit 331a then generates a correction parameter for each control type, by performing a process similar to the process performed by the learning unit 19 in the first arrangement.

For example, the subset generation unit 331b learns a correction parameter when the type of the autonomous control device 10 is a robot, for each control type. For example, the subset generation unit 331c learns a correction parameter when the type of the autonomous control device 10 is a monitoring terminal, for each control type.

As described above, the autonomous control system 100 according to the second arrangement can share the history of the stress information that is determined by the determination device 20 at the time of control performed by the autonomous control devices 10.

The autonomous control system 100 according to the second arrangement can hand over the correction parameter that is learned from the history of the stress information being determined when a certain autonomous control device 10 is controlled, to another autonomous control device 10 of the same kind. For example, even when the user 200 uses a new autonomous control device 10, the new autonomous control device 10 can autonomously control the operation preferred by the user 200, by taking over the correction parameter of the same type of the autonomous control device 10 that has been used by the user 200.

Finally, an example of a hardware configuration of the autonomous control system 100 of the first and second arrangements will be described.

## Hardware Configuration of Autonomous Control Device

FIG. 13 is a diagram illustrating an example of a hardware configuration of the autonomous control device 10 of the first and second arrangements. The autonomous control device 10 of the first and second arrangements includes a control device 301, a main storage device 302, an auxiliary storage device 303, a display device 304, an input device 305, a communication device 306, a sensor 307, and an application specific integrated circuit (ASIC) 308. The control device 301, the main storage device 302, the auxiliary storage device 303, the display device 304, the input device 305, the communication device 306, the sensor 307, and the ASIC 308 are connected via a bus 310.

The control device 301 executes a computer program read out from the auxiliary storage device 303 to the main storage device 302. For example, the control device 301 is a central processing unit (CPU). The main storage device 302 is a memory such as a read-only memory (ROM), and a random-access memory (RAM). The auxiliary storage device 303 is a memory card, a solid state drive (SSD), and the like.

The display device 304 displays information. For example, the display device 304 is a liquid crystal display. The input device 305 receives an input of information. For example, the input device 305 is a button. The display device 304 and the input device 305 may also be a liquid crystal touch panel or the like that has a display function and an input function.

The communication device 306 transmits and receives information to and from the other devices. For example, the communication device 306 is a wireless communication module.

For example, the sensor 307 is a detection device such as the CMOS camera, the millimeter wave radar, and the LIDAR.

The ASIC 308 performs processing on a function that can be implemented by a dedicated circuit, among the functional configurations of the autonomous control device 10 of the first and second arrangements described above. For example, the function that can be implemented by the dedicated circuit is the learning unit 19 (see FIG. 6).

A computer program executed by the autonomous control device 10 of the first and second arrangements is provided as a computer program product by being stored in a computer-readable storage medium such as a compact disc-read only memory (CD-ROM), a memory card, a compact disc-recordable (CD-R), or a digital versatile disc (DVD) in an installable or executable file format.

The computer program executed by the autonomous control device 10 of the first and second arrangements can be stored on a computer connected to a network such as the Internet, and provided by causing a user to download it via the network. The computer program executed by the autonomous control device 10 of the first and second arrangements can also be provided via a network such as the Internet without downloading it.

The computer program executed by the autonomous control device 10 of the first and second arrangements can also be incorporated into the ROM and the like in advance.

The computer program executed by the autonomous control device 10 of the first and second arrangements is composed of a modular configuration including the function that can be implemented by the computer program, among the functional configurations of the autonomous control device 10 of the first and second arrangements described above.

The function implemented by the computer program is loaded on the main storage device 302, when the control device 301 reads and executes the computer program from a storage medium such as the auxiliary storage device 303. In other words, the function implemented by the computer program is generated on the main storage device 302.

Whether a part or all of the functions of the autonomous control device 10 of the first and second arrangements is to be implemented by hardware such as the ASIC 308 or implemented by a computer program executed by the control device 301 can be suitably determined based on the processing speed, the cost, and the like.

## Hardware Configuration of Determination Device

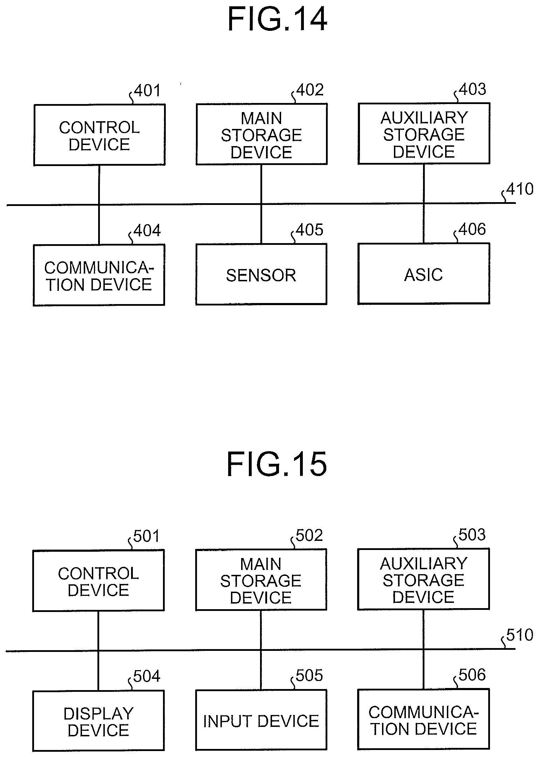

FIG. 14 is a diagram illustrating an example of a hardware configuration of the determination device 20 of the first and second arrangements.

The determination device 20 of the first and second arrangements includes a control device 401, a main storage device 402, an auxiliary storage device 403, a communication device 404, a sensor 405, and an ASIC 406. The control device 401, the main storage device 402, the auxiliary storage device 403, the communication device 404, the sensor 405, and the ASIC 406 are connected via a bus 410.

The control device 401 executes a computer program read out from the auxiliary storage device 403 to the main storage device 402. For example, the control device 401 is a CPU. The main storage device 402 is a memory such as a ROM and a RAM. The auxiliary storage device 403 is a flash memory and the like.

The communication device 404 transmits and receives information to and from the other devices. For example, the communication device 404 is a wireless communication module.

For example, the sensor 405 is a detection device such as the heartbeat sensor 211, the perspiration sensor 212, the body temperature sensor 213, and the odor sensor 214 described above.

The ASIC 406 performs processing on a function that can be implemented by a dedicated circuit, among the functional configurations of the determination device 20 of the first and second arrangements described above. For example, the function that can be implemented by the dedicated circuit is the calculation unit 22 (see FIG. 7).

The computer program executed by the determination device 20 of the first and second arrangements is provided as a computer program product by being stored in a computer-readable storage medium such as a CD-ROM, a memory card, a CD-R, and a DVD in an installable or executable file format.

The computer program executed by the determination device 20 of the first and second arrangements can be stored on a computer connected to a network such as the Internet, and causing a user to download it via the network. The computer program executed by the determination device 20 of the first and second arrangements may also be provided via a network such as the Internet without downloading it.

The computer program executed by the determination device 20 of the first and second arrangements can also be incorporated into the ROM and the like in advance and be provided.

The computer program executed by the determination device 20 of the first and second arrangements is composed of a modular configuration including the function that can be implemented by the computer program, among the functional configurations of the determination device 20 of the first and second arrangements described above.

The function implemented by the computer program is loaded on the main storage device 402 when the control device 401 reads and executes the computer program from a storage medium such as the auxiliary storage device 403. In other words, the function implemented by the computer program is generated on the main storage device 402.

Whether a part or all of the functions of the determination device 20 of the first and second arrangements is to be implemented by hardware such as the ASIC 406 or implemented by a computer program executed by the control device 401 can be suitably determined based on the processing speed, the cost, and the like.

**Hardware Configuration of Server Device**

FIG. 15 is a diagram illustrating an example of a hardware configuration of the server device 30 according to the second arrangement. The server device 30 of the arrangement includes a control device 501, a main storage device 502, an auxiliary storage device 503, a display device 504, an input device 505, and a communication device 506. The control device 501, the main storage device 502, the auxiliary storage device 503, the display device 504, the input device 505, and the communication device 506 are connected via a bus 510.

The control device 501 executes a computer program read out from the auxiliary storage device 503 to the main storage device 502. For example, the control device 501 is a CPU. The main storage device 502 is memory such as a ROM and a RAM. The auxiliary storage device 503 is a memory card, an SSD, and the like.

The input device 505 receives an input of information. The display device 504 displays the information. For example, the display device 504 is a liquid crystal display. For example, the input device 505 is a keyboard and a mouse. The display device 504 and the input device 505 may also be a liquid crystal touch panel that has a display function and an input function. The communication device 506 communicates with the other devices.

The computer program executed by the server device 30 of the arrangement is provided as a computer program product by being stored in a computer-readable storage medium such as a CD-ROM, a memory card, a CD-R, or a DVD in an installable or executable file format.

The computer program executed by the server device 30 of the arrangement can be stored on a computer connected to a network such as the Internet, and provided by causing a user to download it via the network. The computer program executed by the server device 30 of the arrangement can also be provided via a network such as the Internet without downloading it.

The computer program executed by the server device 30 of the arrangement can also be incorporated into the ROM and the like in advance and be provided.

The computer program executed by the server device 30 of the arrangement is composed of a modular configuration including the function that can be implemented by the computer program, among the functional configurations of the server device 30 of the arrangement described above.

The function implemented by the computer program is loaded on the main storage device 502 when the control device 501 reads and executes the computer program from a storage medium such as the auxiliary storage device 503. In other words, the function implemented by the computer program is generated on the main storage device 502.

A part or all of the functions of the server device 30 of the arrangement can also be implemented by hardware such as an integrated circuit (IC).

While certain arrangements have been described, these arrangements have been presented by way of example only, and are not intended to limit the scope of the claims. Indeed, the apparatuses described herein may be embodied in a variety of other forms; furthermore various omissions, substitutions and changes in the form of the apparatuses described herein may be made.

**Example 1.** An autonomous control system includes a first detecting unit, an identifying unit, a first determining unit, a learning unit, a second determining unit, and a controlling unit. The first detecting unit detects surrounding information of an object to be controlled. The identifying unit identifies identification information indicating an object to be identified from the surrounding information. The first determining unit determines an increase and decrease in stress information indicating a degree of stress of a user, from biological information of the user. The learning unit learns correction information for correcting an operation of the object to be controlled, to an operation of reducing the stress of the user that is indicated by the stress information, from the increase and decrease in the stress information. The second determining unit determines a type of control relative to the object to be controlled, and determines control information for specifying the operation of the object to be controlled by the determined type of control, from the identification information and the correction information. The controlling unit controls the object to be controlled by the control information.

**Example 2.** In the autonomous control system according to Example 1, the first determining unit determines the increase and decrease in the stress information during an inquiry period that is specified according to the type of control.

**Example 3.** The autonomous control system according to Example 1, further includes: a second detecting unit that detects the biological information of the user, from at least one of a heartbeat sensor, a perspiration sensor, a body temperature sensor, and an odor sensor.

**Example 4.** The autonomous control system according to Example 3, further including: a power feeding unit that supplies power to the processing circuitry, using energy harvesting.

**Example 5.** In the autonomous control system according to Example 1, the correction information is a correction parameter including a plurality of parameters; and the learning unit learns the correction parameter such that a degree of unpleasantness indicated by the stress information that is determined after a part or all of the parameters is changed is decreased.

**Example 6.** A server device includes a first communicating unit, a second communicating unit, a storage unit, and a learning unit. The first communicating unit receives first reception data in which stress information indicating a degree of stress determined from biological information of a user, and determination time indicating time when the stress information is determined are associated, from a determination device. The second communicating unit receives second reception data in which a device type indicating a type of a first autonomous control device, a control type indicating a type of control, and a control time indicating time when the control is performed are associated, from the first autonomous control device. The storage unit stores in the memory, the stress information included in the first reception data in which a difference between the determination time and the control time is equal to or less than a threshold, and the device type and the control type that are included in the second reception data in which a difference between the determination time and the control time is equal to or less than the threshold, in an associated manner. The learning unit learns correction information for correcting an operation of an object to be controlled to an operation preferred by the user, from history of the stress information, for each combination of the device type and the control type. The second communicating unit transmits the correction information corresponding to a combination of the device type of a second autonomous control device and the control type of control by the second autonomous control device, to the second autonomous control device.

**Example 7.** In the server device according to Example 6, the correction information is a correction parameter including a plurality of parameters, and the learning unit learns the correction parameter such that a degree of unpleasantness indicated by the stress information that is determined after a part or all of the parameters is changed is decreased, for each combination of the device type and the control type.

**Example 8.** An autonomous control method includes: detecting, by a first detecting unit, surrounding information of an object to be controlled; identifying, by an identifying unit, identification information indicating an object to be identified from the surrounding information; determining, by a first determining unit, an increase and decrease in stress information indicating a degree of stress of a user, from biological information of the user; learning, by a learning unit, correction information for correcting an operation of the object to be controlled, to an operation of reducing the stress of the user indicated by the stress information, from the increase and decrease in the stress information; determining, by a second determining unit, a type of control relative to the object to be controlled, from the identification information and the correction information; determining, by a third determining unit, control information for specifying the operation of the object to be controlled by the determined type of control; and controlling, by a controlling unit, the object to be controlled by the control information.

**Example 9.** In the autonomous control method according to Example 8, the determining of the increase and decrease in the stress information includes determining the increase and decrease in the stress information during an inquiry period that is specified according to the type of control.

**Example 10.** The autonomous control method according to Example 8, further including: detecting, by a second detecting unit, the biological information of the user, from at least one of a heartbeat sensor, a perspiration sensor, a body temperature sensor, and an odor sensor.

**Example 11.** The autonomous control method according to Example 10, further including: suppling, by a power feeding unit, power to the processing circuitry, using energy harvesting.

**Example 12.** In the autonomous control method according to Example 8, the correction information is a correction parameter including a plurality of parameters, and the learning includes learning the correction parameter such that a degree of unpleasantness indicated by the stress information that is determined after a part or all of the parameters is changed is decreased.

## Claims

1. An autonomous control system (100), comprising:

   a first detecting unit (11) that detects surrounding information of an object to be controlled;
   an identifying unit (12) that identifies identification information indicating an object to be identified from the surrounding information;
   a first determining unit (24) that determines an increase and decrease in stress information indicating a degree of stress of a user, from biological information of the user;
   a learning unit (19) that learns correction information for correcting an operation of the object to be controlled, to an operation of reducing the stress of the user that is indicated by the stress information, from the increase and decrease in the stress information;
   a second determining unit (13) that determines a type of control relative to the object to be controlled, and determines control information for specifying the operation of the object to be controlled by the determined type of control, from the identification information and the correction information; and
   a controlling unit (14) that controls the object to be controlled by the control information.

2. The autonomous control system (100) according to claim 1, wherein the first determining unit (24) determines the increase and decrease in the stress information during an inquiry period that is specified according to the type of control.

3. The autonomous control system (100) according to claim 1, further comprising:

   a second detecting unit (21) that detects the biological information of the user, from at least one of a heartbeat sensor, a perspiration sensor, a body temperature sensor, and an odor sensor.

4. The autonomous control system (100) according to claim 3, further comprising:

   a power feeding unit (26) that supplies power to the processing circuitry, using energy harvesting.

5. The autonomous control system (100) according to claim 1, wherein
   the correction information is a correction parameter including a plurality of parameters; and
   the learning unit (19) learns the correction parameter such that a degree of unpleasantness indicated by the stress information that is determined after a part or all of the parameters is changed is decreased.

6. A server device (30), comprising:

   a first communicating unit (31a) that receives first reception data in which stress information indicating a degree of stress determined from biological information of a user, and determination time indicating time when the stress information is determined are associated, from a determination device;
   a second communicating unit (31b) that receives second reception data in which a device type indicating a type of a first autonomous control device, a control type indicating a type of control, and a control time indicating time when the control is performed are associated, from the first autonomous control device;
   a storage unit (32) that stores in the memory, the stress information included in the first reception data in which a difference between the determination time and the control time is equal to or less than a threshold, and the device type and the control type that are included in the second reception data in which a difference between the determination time and the control time is equal to or less than the threshold, in an associated manner; and a learning unit (33) that learns correction information for correcting an operation of an object to be controlled to an operation preferred by the user, from history of the stress information, for each combination of the device type and the control type, wherein

the second communicating unit (31b) transmits the correction information corresponding to a combination of the device type of a second autonomous control device and the control type of control by the second autonomous control device, to the second autonomous control device.

7. The server device (30) according to claim 6, wherein

the correction information is a correction parameter including a plurality of parameters; and the learning unit (33) learns the correction parameter such that a degree of unpleasantness indicated by the stress information that is determined after a part or all of the parameters is changed is decreased, for each combination of the device type and the control type.

8. An autonomous control method, comprising:

detecting, by a first detecting unit (11), surrounding information of an object to be controlled; identifying, by an identifying unit (12), identification information indicating an object to be identified from the surrounding information;

determining, by a first determining unit (24), an increase and decrease in stress information indicating a degree of stress of a user, from biological information of the user;

learning, by a learning unit (19), correction information for correcting an operation of the object to be controlled, to an operation of reducing the stress of the user indicated by the stress information, from the increase and decrease in the stress information;

determining, by a second determining unit (13), a type of control relative to the object to be controlled, from the identification information and the correction information;

determining, by the second determining unit (13), control information for specifying the operation of the object to be controlled by the determined type of control; and

controlling, by a controlling unit (14), the object to be controlled by the control information.

9. The autonomous control method according to claim 8, wherein the determining of the increase and decrease in the stress information includes determining the increase and decrease in the stress information during an inquiry period that is specified according to the type of control.

10. The autonomous control method according to claim 8, further comprising: detecting, by a second detecting unit (21), the biological information of the user, from at least one of a heartbeat sensor, a perspiration sensor, a body temperature sensor, and an odor sensor.

11. The autonomous control method according to claim 10, further comprising: suppling, by a power feeding unit (26), power to the processing circuitry, using energy harvesting.

12. The autonomous control method according to claim 8, wherein

the correction information is a correction parameter including a plurality of parameters; and the learning includes learning the correction parameter such that a degree of unpleasantness indicated by the stress information that is determined after a part or all of the parameters is changed is decreased.

# FIG.1

100

200

10

| 11 DETEC-TION UNIT | → | 12 IDENTIFI-CATION UNIT | → | 13 DETER-MINATION UNIT | → | 14 CONTROL UNIT | → | 15 OUTPUT UNIT |

19 LEARNING UNIT

CONTROL TYPE

INQUIRY PERIOD

18 STORAGE UNIT

STRESS INFORMATION INCREASE AND DECREASE

17 COMMUNI-CATION UNIT

16 INQUIRY UNIT

20

25 COMMUNI-CATION UNIT

21 DETEC-TION UNIT

24 DETER-MINATION UNIT

22 CALCULA-TION UNIT

26 POWER FEEDING UNIT

23 STORAGE UNIT

# FIG.2

| TRANSMISSION DESTINATION ADDRESS | TRANSMISSION SOURCE ADDRESS | INQUIRY NUMBER | INQUIRY PERIOD | FCS |
|---|---|---|---|---|

# FIG.3

| TRANSMISSION DESTINATION ADDRESS | TRANSMISSION SOURCE ADDRESS | INQUIRY NUMBER | STRESS INFORMATION INCREASE AND DECREASE | FCS |
|---|---|---|---|---|

# FIG.4

# FIG.5A

| CONTROL TYPE | CONTROLLED NUMBER OF TIMES | STRESS INFORMATION INCREASE AND DECREASE (NUMBER OF TIMES) |
|---|---|---|
| ADVANCING STRAIGHT AT YELLOW TRAFFIC LIGHT | 8 | 4 |
| TURNING RIGHT AT YELLOW TRAFFIC LIGHT | 6 | 4 |
| OVERTAKING | 4 | 2 |
| PARKING | 12 | 2 |

# FIG.5B

| CONTROL TYPE | CONTROLLED NUMBER OF TIMES | STRESS INFORMATION INCREASE AND DECREASE (ACCUMULATED VALUE) |
|---|---|---|
| ADVANCING STRAIGHT AT YELLOW TRAFFIC LIGHT | 8 | 12 |
| TURNING RIGHT AT YELLOW TRAFFIC LIGHT | 6 | 16 |
| OVERTAKING | 4 | 4 |
| PARKING | 12 | 2 |

# FIG.5C

| CONTROL TYPE | CONTROLLED NUMBER OF TIMES | STRESS INFORMATION INCREASE AND DECREASE (INTEGRATED VALUE) |
|---|---|---|
| ADVANCING STRAIGHT AT YELLOW TRAFFIC LIGHT | 8 | 300 |
| TURNING RIGHT AT YELLOW TRAFFIC LIGHT | 6 | 400 |
| OVERTAKING | 4 | 100 |
| PARKING | 12 | 200 |

# FIG.6

# FIG.7

# FIG.8

START

| |
|---|
| **S1** |
| SET INITIAL VALUE OF CORRECTION PARAMETER |

| |
|---|
| **S2** |
| INITIALIZE HISTORY OF STRESS INFORMATION INCREASE AND DECREASE |

| |
|---|
| **S3** |
| n=0,1,···,N |

| |
|---|
| **S4** |
| SENSE |

| |
|---|
| **S5** |
| IDENTIFY |

| |
|---|
| **S6** |
| DETERMINE |

| |
|---|
| **S7** |
| CONTROL |

| |
|---|
| **S8** |
| INQUIRE STRESS INFORMATION INCREASE AND DECREASE |

| |
|---|
| **S9** |
| ACCUMULATE STRESS INFORMATION INCREASE AND DECREASE |

| |
|---|
| **S10** |
| UPDATE CORRECTION PARAMETER |

# FIG.9

# FIG.10

# FIG.11

| TRANSMISSION DESTINATION ADDRESS | TRANSMISSION SOURCE ADDRESS | CONTROL TIME | DEVICE TYPE | CONTROL TYPE | FCS |
|---|---|---|---|---|---|

# FIG.12

| TRANSMISSION DESTINATION ADDRESS | TRANSMISSION SOURCE ADDRESS | DETERMINA-TION TIME | STRESS INFORMA-TION | FCS |
|---|---|---|---|---|

# FIG.13

| CONTROL DEVICE 301 | MAIN STORAGE DEVICE 302 | AUXILIARY STORAGE DEVICE 303 | DISPLAY DEVICE 304 |
|---|---|---|---|
| INPUT DEVICE 305 | COMMUNICA-TION DEVICE 306 | SENSOR 307 | ASIC 308 |

310

# FIG.14

| 401 CONTROL DEVICE | 402 MAIN STORAGE DEVICE | 403 AUXILIARY STORAGE DEVICE |
|---|---|---|

— 410

| 404 COMMUNICA- TION DEVICE | 405 SENSOR | 406 ASIC |
|---|---|---|

# FIG.15

| 501 CONTROL DEVICE | 502 MAIN STORAGE DEVICE | 503 AUXILIARY STORAGE DEVICE |
|---|---|---|

— 510

| 504 DISPLAY DEVICE | 505 INPUT DEVICE | 506 COMMUNICA- TION DEVICE |
|---|---|---|

Europäisches
Patentamt

European
Patent Office

Office européen
des brevets

# EUROPEAN SEARCH REPORT

Application Number

EP 17 15 8182

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP 2016 052881 A (DENSO CORP) 14 April 2016 (2016-04-14) * abstract; figures 1-14,28-30 * | 1-12 | INV. B60W50/00 B60W40/08 |
| E | & US 2017/225677 A1 (YOSHIDA ICHIRO [JP] ET AL) 10 August 2017 (2017-08-10) * abstract; figures 1-14,28-30 * * paragraphs [0001] - [0008], [0045] - [0102], [0125], [0155] - [157162], [0171], [0175], [0177], [0178], [0184] * | 1,3-8, 10-12 | A61B5/00 B60W50/14 |
| X | US 2015/246673 A1 (TSENG FLING [US] ET AL) 3 September 2015 (2015-09-03) * abstract; figures 1,2 * * paragraphs [0004], [0005], [0009] - [0024] * | 1-12 | |
| X | US 2015/243172 A1 (ESKILSON ANDERS [SE]) 27 August 2015 (2015-08-27) * abstract; figure 3 * * paragraphs [0001], [0004], [0008], [0015], [0017], [0019], [0035], [0036], [0043] * | 1-12 | TECHNICAL FIELDS SEARCHED (IPC) B60W A61B |
| A | WO 2016/033587 A1 (IMS SOLUTIONS INC [US]) 3 March 2016 (2016-03-03) * paragraphs [0006], [0007] * | 3 | |

The present search report has been drawn up for all claims

| Place of search | Date of completion of the search | Examiner |
|---|---|---|
| Munich | 26 October 2017 | Elbel, Benedikte |

EPO FORM 1503 03.82 (P04C01)

## ANNEX TO THE EUROPEAN SEARCH REPORT
## ON EUROPEAN PATENT APPLICATION NO.

EP 17 15 8182

This annex lists the patent family members relating to the patent documents cited in the above-mentioned European search report.
The members are as contained in the European Patent Office EDP file on
The European Patent Office is in no way liable for these particulars which are merely given for the purpose of information.

26-10-2017

| Patent document cited in search report | | Publication date | Patent family member(s) | | Publication date |
|---|---|---|---|---|---|
| JP 2016052881 | A | 14-04-2016 | JP 2016052881 A | | 14-04-2016 |
| | | | US 2017225677 A1 | | 10-08-2017 |
| US 2015246673 | A1 | 03-09-2015 | CN 104875746 A | | 02-09-2015 |
| | | | DE 102015203354 A1 | | 03-09-2015 |
| | | | GB 2525079 A | | 14-10-2015 |
| | | | MX 347369 B | | 25-04-2017 |
| | | | RU 2015106806 A | | 20-09-2016 |
| | | | US 2015246673 A1 | | 03-09-2015 |
| US 2015243172 | A1 | 27-08-2015 | EP 2898385 A1 | | 29-07-2015 |
| | | | KR 20150055052 A | | 20-05-2015 |
| | | | KR 20170037681 A | | 04-04-2017 |
| | | | SE 1251072 A1 | | 25-03-2014 |
| | | | US 2015243172 A1 | | 27-08-2015 |
| | | | WO 2014046602 A1 | | 27-03-2014 |
| WO 2016033587 | A1 | 03-03-2016 | US 2017247037 A1 | | 31-08-2017 |
| | | | WO 2016033587 A1 | | 03-03-2016 |

EPO FORM P0459

For more details about this annex : see Official Journal of the European Patent Office, No. 12/82